# EUROPEAN PATENT APPLICATION

(11) **EP 4 595 993 A1**
(43) Date of publication of application: **06.08.2025**
(21) Application number: 23870915.8
(22) Date of filing: 27.09.2023
(51) Int. Cl.: A61M 1/36, A61M 1/34, A61B 17/34, A61F 2/95

(54) **EXTRACORPOREAL CIRCULATION SYSTEM**

(30) Priority: 29.09.2022 CN 202222620086 U; 29.09.2022 CN 202211201983
(71) Applicant: Shenzhen Wecan Medical Technology Co., Ltd., Shenzhen, Guangdong 518063 (CN)
(72) Inventor: WU, Liping, Shenzhen, Guangdong 518000 (CN); LI, Zhen, Shenzhen, Guangdong 518000 (CN); ZHANG, Xiong, Shenzhen, Guangdong 518000 (CN); FENG, Haoyuan, Shenzhen, Guangdong 518000 (CN)
(74) Representative: Michalski Hüttermann & Partner Patentanwälte mbB
(86) International application number: PCT/CN2023/121977
(87) International publication number: WO 2024/067688

(57) **Abstract**

Provided is an extracorporeal circulation system, which includes an arterial sheath, an inflow tube, a handle, an outflow tube, and a venous sheath that are connected in sequence. The arterial sheath is used for connecting to a carotid artery, and the venous sheath is used for connecting to a vein. The handle includes a housing, as well as a flow controller, a blood filter, and a one-way check valve that are arranged in the housing and are communicated in sequence. The flow controller is connected to the end of the inflow tube away from the arterial sheath, and the one-way check valve is connected to the end of the outflow tube away from the venous sheath. The flow controller includes one body element with a flow-through channel and a flow control element. At least part of the flow control element is received in the body element and is movably connected to the flow-through channel. The flow control element reduces or increases the circulation area of the flow-through channel under an external force. Reverse blood flow from the carotid artery to the femoral vein can be established by using the extracorporeal circulation system of the present invention, thereby preventing a falling thrombus from entering the brain during surgery.

## Description

### Technical Field

The present invention relates to the technical field of medical instruments, and in particular, to an extracorporeal circulation system.

### Background Art

Carotid artery disease generally occurs when thrombi and blood clots deposit in side walls of the common and internal carotid arteries (through which the blood flows from the heart to the brain), which narrow the arteries. These deposits easily fall off and form emboli flowing with the blood. When entering the brain with the blood through the carotid artery, thrombi will cause a stroke.

Among the surgical procedures for treating carotid artery stenosis, carotid endarterectomy (CEA) is the standard surgical procedure. The specific procedure includes: a relatively large surgical incision is formed on the neck of a patient, an upstream and a downstream of a surgical site on a carotid artery are respectively clamped to block blood flow, the carotid artery is cut open to remove deposits, the carotid artery is sutured after the deposits are removed, and the circulation in the carotid artery is restored. This surgical procedure causes a large incision and is likely to damage cranial nerves near the carotid artery. In addition, there is still a high probability of postoperative stroke. Carotid artery stenting (CAS) is a minimally invasive surgical procedure for treating carotid artery stenosis. The specific procedure includes: a delivery sheath is introduced into the femoral artery through percutaneous puncture, and then ascends along the aortic arch into a carotid artery, and a self-expanding stent is released in the carotid artery to restore normal circulation in the carotid artery. However, during CAS, the deployment of the delivery sheath and the expansion of the self-expanding stent are likely to cause deposits to be dislodged off an inner wall of the carotid artery. The probability of postoperative stroke is much higher than that of CEA, and there are obvious shortcomings in its clinical use.

### Summary of the Invention

Based on this, it is necessary to provide a medical apparatus for treating carotid artery stenosis that reduces the risk of cerebral stroke and results in only a small incision.

The present invention provides an extracorporeal circulation system, which includes an arterial sheath, an inflow tube, a handle, an outflow tube, and a venous sheath that are connected in sequence. The arterial sheath is used for connecting to a carotid artery, and the venous sheath is used for connecting to a vein.

The handle includes a housing, as well as a flow controller, a blood filter, and a one-way check valve that are arranged in the housing and are communicated in sequence. The flow controller is connected to the end of the inflow tube that is away from the arterial sheath, and the one-way check valve is connected to the end of the outflow tube that is away from the venous sheath.

The flow controller includes one body element with a flow-through channel and a flow control element. At least part of the flow control element is received in the body element and is movably connected to the flow-through channel. The flow control element reduces or increases a circulation area of the flow-through channel under an external force.

In an embodiment, the flow-through channel has an inflow end and an outflow end. The inflow end is close to the inflow tube, and the outflow end is far away from the inflow tube.

The flow control element has one flow guiding surface. The flow guiding surface faces the inflow end. One flow regulating channel gradually decreasing from the inflow end to the outflow end is enclosed by the flow guiding surface and an inner surface of the flow-through channel.

In an embodiment, a moving channel communicated with the flow-through channel is further arranged in the body element.

The flow control element is movably arranged in the moving channel.

In an embodiment, the flow-through channel includes a connecting section, a narrowed section, and a body section that are connected in sequence from the inflow end to the outflow end. An inner diameter of the narrowed section is smaller than the inner diameters of any one of the connecting section and the body section.

In an embodiment, one inclined surface is formed at the distal end of the flow control element and defines the flow guiding surface.

In an embodiment, the distal end surface of the flow control element is matched with the shape of the bottom of the flow-through channel. When the flow controller is in an off state, the distal end surface of the flow control element fits the bottom of the flow-through channel.

In an embodiment, the flow control element includes a rotary knob and a sliding piece. The sliding piece is slidably arranged in the moving channel. The rotary knob is rotatably connected to the body element and is in threaded connection with the sliding piece. The rotary knob rotates under an external force and drives the sliding piece to spirally move in the moving channel.

The inclined surface is arranged at a distal end of the sliding piece.

In an embodiment, the included angle between the central axis of the flow-through channel and the central axis of the moving channel is an acute angle, the moving channel is inclined toward the inflow end of the body element, and a partial peripheral surface of the flow control element forms the flow guiding surface.

In an embodiment, a terminal of the flow control element is in the shape of a ball head, and the diameter of the terminal in the shape of the ball head is equal to the inner diameter of the flow-through channel.

A plurality of communication holes is formed in the flow control element, and the communication holes extend in the same direction as the flow-through channel. At least one communication hole includes a velocity regulating section, the entire velocity regulating section is in the shape of a hopper gradually decreasing from the inflow end to the outflow end, and an inner wall of the velocity regulating section forms the flow guiding surface.

An edge of the inflow end of the velocity regulating section is in smooth transition connection with an inner wall of the flow-through channel.

In an embodiment, a plurality of first matching portions are arranged on the body element, a plurality of second matching portions are arranged on the flow control element, and when any first matching portion is matched with the second matching portion, the edge of the inflow end of the corresponding velocity regulating section is in smooth transition connection with the inner wall of the flow-through channel.

In an embodiment, a moving channel communicating with the flow-through channel is further arranged in the body element, the flow control element is rotatably arranged in the moving channel, and a circulation hole is formed in the flow control element and communicates with the flow-through channel.

In an embodiment, a first limiting piece and a second limiting piece are arranged outside the housing; when the flow control element abuts against the first limiting piece, the flow controller is in an off state; and when the flow control element abuts against the second limiting piece, the flow controller is in a maximum flow state.

In an embodiment, the flow control element abuts against the bottom surface of the moving channel.

In an embodiment, a window is formed in a side surface of the housing, and the flow control element is mainly arranged in the housing.

A handle is arranged on the flow control element and is exposed outside the housing from the window, and when the handle abuts against one side of two sides of the window, the flow controller is in an off state or a maximum flow state.

In an embodiment, the filter includes a filter element and a filter screen arranged in the filter element. The filter element includes a tubular structure with openings at two ends, and a gap is provided between the filter screen and the filter element. The filter screen includes a semi-closed structure with an opening at one end, and the opening of the filter screen is connected to the outflow end of the flow controller.

In an embodiment, the circumference of the minimum cross section of the filter screen is at least twice the circumference of the cross section of the flow-through channel.

In an embodiment, the filter includes a filter element and at least one sheet-like filter screen arranged in the filter element. The filter element includes a tubular structure with openings at two ends, and the sheet-like filter screen is perpendicular to the central axis of the filter element.

In an embodiment, the inflow tube and the outflow tube are made from a thermal insulation material, or the exterior of the inflow tube and the exterior of the outflow tube are covered with a thermal insulation layer, or a heating apparatus is arranged at the outflow tube or the venous sheath.

In an embodiment, the blood filter has a filter outlet channel, and the filter outlet channel is connected to the one-way check valve. The inner diameter of the filter outlet channel is denoted as d, the inner diameter of the blood filter is denoted as D, and d≤1/4D.

A reverse blood flow channel from the carotid artery to the femoral vein can be established by using the extracorporeal circulation system of the present invention. Before a stent is placed at a stenotic site of the carotid artery, the arterial sheath of the present invention is placed in the common carotid artery and the venous sheath is placed in the femoral vein. A pressure difference is formed between the high-pressure arterial blood flow in the brain and the low-pressure venous blood flow in the femoral vein, so that the blood in the carotid artery flows in reverse to the femoral vein through the extracorporeal circulation system. After the reverse blood circulation system is established, operations, such as pre-expansion of the stenotic site of the carotid artery and placement of a self-expanding stent, are performed. Any thrombus falling off during surgery flows with the reverse blood flow and is intercepted by the blood filter, so that the thrombus is prevented from entering the human body with blood flow. A doctor can regulate the flow rate of the reverse blood flow in real time by using the flow controller.

### Brief Description of the Drawings

FIG. 1 is a top view of an extracorporeal circulation system according to Embodiment 1 of the present invention.
FIG. 2 is a cross-sectional view taken along line A in FIG. 1.
FIG. 3 is a structural exploded view of a flow controller of Embodiment 1 of the present invention.
FIG. 4 is a cross-sectional view of the flow controller of Embodiment 1 of the present invention (the flow controller is in a partial circulation sate).
FIG. 5 is a cross-sectional view of the flow controller of Embodiment 1 of the present invention (the flow controller is in an off state).
FIG. 6 is a structural exploded view of a flow controller of Embodiment 2 of the present invention.
FIG. 7 is a cross-sectional view of the flow controller of Embodiment 2 of the present invention (the flow controller is in a partial circulation state).
FIG. 8 is a cross-sectional view of the flow controller of Embodiment 2 of the present invention (the flow controller is in an off state).
FIG. 9 is a stereogram of a flow controller of Embodiment 3 of the present invention.
FIG. 10 is a cross-sectional view of the flow controller of Embodiment 3 of the present invention (the flow controller is in an off state).
FIG. 11 is a cross-sectional view of the flow controller of Embodiment 3 of the present invention (the flow controller is in a partial circulation state).
FIG. 12 is a top view of an extracorporeal circulation system of Embodiment 4 of the present invention.
FIG. 13 is a cross-sectional view taken along line B in FIG. 12.
FIG. 14 is a schematic structural diagram of a filter of another embodiment.
FIG. 15 is a front view of an extracorporeal circulation system of Embodiment 5 of the present invention.
FIG. 16 is a top view of an extracorporeal circulation system of Embodiment 5 of the present invention (a flow controller in a maximum flow state).
FIG. 17 is a cross-sectional view taken along line C in FIG. 16.

### Detailed Description of the Invention

To make the objectives, the technical solutions, and advantages of the present invention clearer, the present invention is further described in detail below with reference to the accompanying drawings and embodiments. It should be understood that the described specific embodiments herein are merely used for explaining the present invention rather than limiting the present invention.

Unless otherwise specified, all technical and scientific terms used herein have the same meaning as commonly understood by those of ordinary skill in the art to which the present invention belongs. The terms used in the description of the present invention herein are for the purpose of describing specific embodiments only and are not intended to limit the present invention. As used herein, the term "and/or" includes any and all combinations of one or more of the associated listed items.

To more clearly describe a structure of an extracorporeal circulation system, the terms "distal end" and "proximal end" are defined herein as conventional terms in the field of interventional medical devices. Specifically, the "distal end" refers to an end away from an operator during surgery, and the "proximal end" refers to an end close to the operator during surgery.

### Embodiment 1

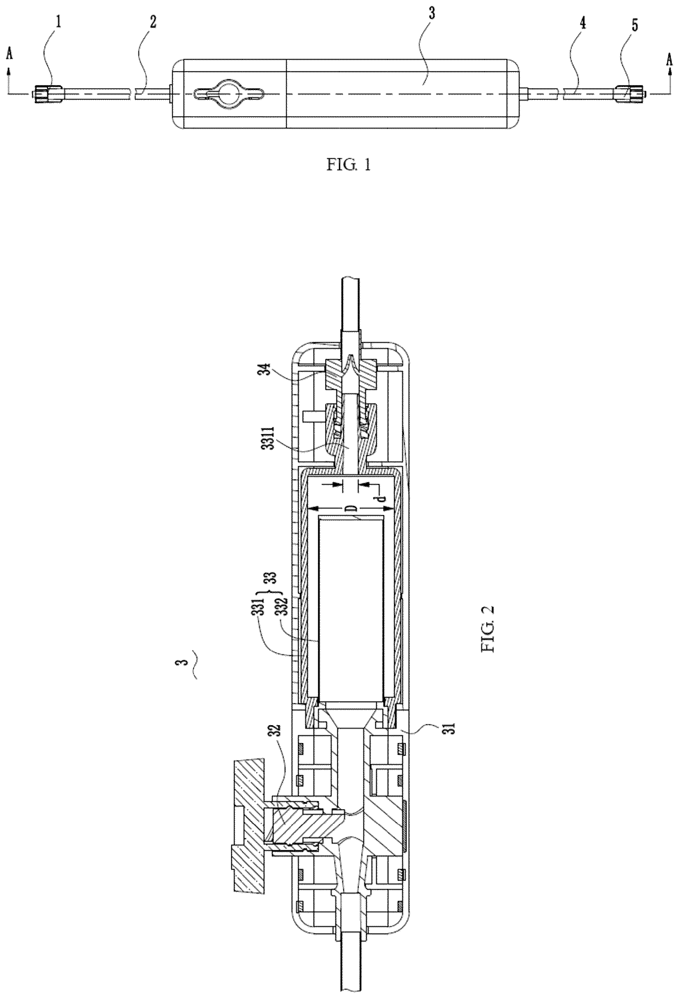

As shown in FIG. 1 and FIG. 2, an extracorporeal circulation system of the present invention includes an arterial sheath 1, an inflow tube 2, a handle 3, an outflow tube 4, and a venous sheath 5. The arterial sheath 1 is used for connecting to a carotid artery, and the venous sheath 5 is used for connecting to a vein.

The handle 3 includes a housing 31, as well as a flow controller 32, a blood filter 33, and a one-way check valve 34 that are arranged in the housing 31 and are communicated in sequence. The flow controller 32 is connected to an end of the inflow tube 2 that is away from the arterial sheath 1, and the one-way check valve 34 is connected to the end of the outflow tube that is away from the venous sheath 5.

The flow controller 32 includes one body element 321 with a flow-through channel 3211 and a flow control element 322. At least part of the flow control element 322 is received in the body element 321 and is movably connected to the flow-through channel 3211. The flow control element 322 reduces or increases a circulation area of the flow-through channel 3211.

Before a stent is placed at a stenotic site of the carotid artery, the arterial sheath 1 of the present invention is placed in the common carotid artery and the venous sheath 5 is placed in the femoral vein. A pressure difference is formed between the high-pressure arterial blood flow in the brain and the low-pressure venous blood flow in the femoral vein, so that the blood in the carotid artery flows in reverse to the femoral vein through the extracorporeal circulation system. After the reverse blood circulation system is established, operations, such as pre-expansion of the stenotic site of the carotid artery and placement of a self-expanding stent, are performed. Any thrombus falling off during surgery flows with the reverse blood flow and is intercepted by the blood filter, so that the thrombus is prevented from entering the human body with the blood flow. A doctor can regulate a flow rate of the reverse blood flow in real time by using the flow controller 32.

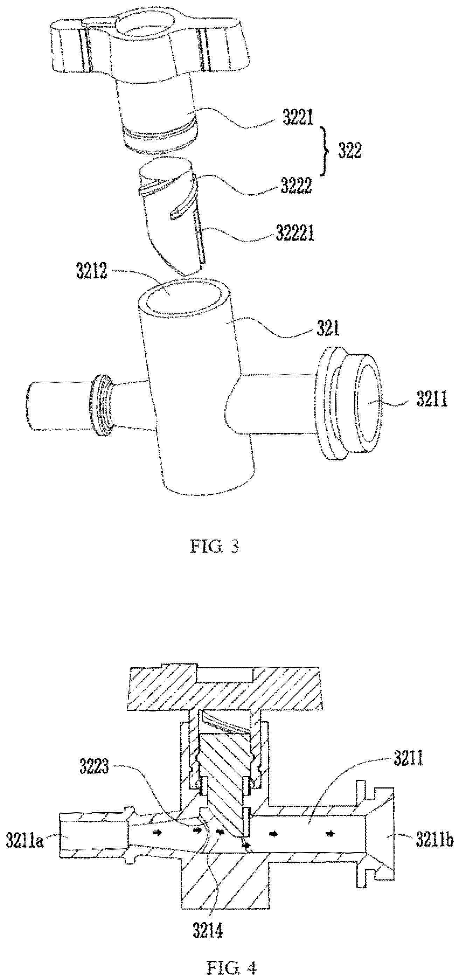

In this embodiment, as shown in FIG. 3 and FIG. 4, a moving channel 3212 communicated with the flow-through channel 3211 is further arranged in the body element 321. The flow control element 322 is movably arranged in the moving channel 3212. Under an external force, a distal end of the flow control element 322 moves toward the flow-through channel 3211. The distal end of the flow control element 322 exceeds the moving channel 3212 and enters the flow-through channel 3211, to obstruct the flow-through channel 3211 to a certain extent, so that the circulation area of the flow-through channel 3211 is reduced. Therefore, the flow rate of the extracorporeal circulation system is reduced. On the contrary, under an external force, the distal end of the flow control element 322 moves away from the flow-through channel 3211, to gradually release the obstruction to the flow-through channel 3211, so that the flow rate of the extracorporeal circulation system is increased. In this way, a flow velocity of the blood can be regulated steplessly to avoid causing discomfort to the human body.

In other embodiments, the moving channel is not arranged on the body element, but a through hole is formed in a side wall of the flow-through channel. The flow control element extends through the through hole and moves along the through hole, to regulate the circulation area of the flow-through channel.

For ease of description, the inflow end 3211a and the outflow end 3211b of the flow-through channel 3211 are shown in FIG. 4. The inflow end 3211a is close to the inflow tube 2, and the outflow end 3211b is far away from the inflow tube 2. In practice, the blood flows into the flow-through channel 3211 from the inflow end 3211a and flows out of the outflow end 3211b. An arrow in FIG. 4 indicates a blood flow direction.

The flow control element 322 has one flow guiding surface 3223. The flow guiding surface is an inclined surface or curved surface, and faces the inflow end 3211a. One flow regulating channel 3214 gradually decreasing from the inflow end 3211a to the outflow end 3211b is enclosed by the flow guiding surface and an inner surface of the flow-through channel 3211.

In this embodiment, one inclined surface is formed at the distal end of the flow control element 322 and forms the flow guiding surface 3223. Because the extracorporeal circulation system of the present invention is used in combination with carotid artery stenting (CAS), the thrombus in the carotid artery will inevitably fall off and flow through the extracorporeal circulation system during surgery. Due to the presence of the thrombus, any obstruction or bending of the flow-through channel 3211 will increase the risk of thrombus deposits, thereby creating the risk of blocking the flow controller 32.

Therefore, the flow control element 322 of the present invention has the flow guiding surface 3223, so that it is difficult for thrombi to deposit on the flow guiding surface inclined relative to the blood flow direction. Even if the flow controller 32 is in a small flow state, it is also difficult for the thrombi to deposit at the flow controller 32 to form a large thrombus. In this way, the unblocked extracorporeal circulation system is ensured.

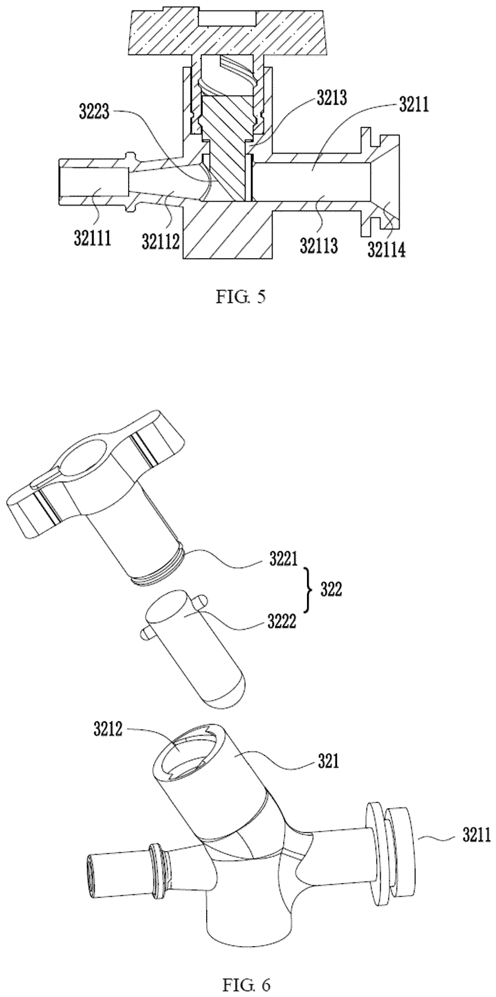

As shown in FIG. 5, the flow-through channel 3211 includes a connecting section 32111, a narrowed section 32112, and a body section 32113 that are connected in sequence from the inflow end 3211a to the outflow end 3211b. The moving channel 3212 is arranged on the body section 32113 and close to the narrowed section 32112. The inner diameter of the narrowed section 32112 is smaller than the inner diameters of any one of the connecting section 32111 and the body section 32113. The connecting section 32111 is connected to the inflow tube 2, and the narrowed section 32112 with a small inner diameter is connected behind the connecting section 32111. When the blood flows through the narrowed section 32112, the velocity is increased, and the blood flows through the flow regulating channel 3214 at a higher velocity, thereby further avoiding deposits of thrombi on an inflow side of the flow control element 322.

As shown in FIG. 5, the distal end surface of the flow control element 322 is matched with a shape of the bottom of the flow-through channel 3211. Therefore, the distal end of the flow control element 322 can closely fit the flow-through channel 3211. That is, when in an off state, the flow controller 32 can exert the function of blocking the blood flow.

Specifically, in this embodiment, as shown in FIG. 3 and FIG. 5, a sliding slot 32221 is formed in a side surface of a sliding piece 3222, and a bump 3213 is arranged in the moving channel 3212 and slidably cooperates with the sliding slot 32221, so that the sliding piece 3222 can move in the moving channel 3212 in an axial direction. An annular groove is formed in an outer wall of a rotary knob 3221, and a protrusion plugged into the annular groove is formed on an inner wall of the moving channel 3212, so that the rotary knob 3221 can rotate along the moving channel 3212 but cannot move in the axial direction. A receiving cavity is formed at a distal end of the rotary knob 3221, and the sliding piece 3222 is inserted into the cavity and is in threaded connection with the rotary knob 3221. The sliding piece 3222 slidably cooperates with the moving channel 3212. When the rotary knob 3221 rotates under an external force, the sliding piece 3222 in threaded connection with the rotary knob 3221 can only move in the axial direction of the moving channel 3212, thereby ensuring that the flow guiding surface always faces the inflow end 3211a of the flow-through channel 3211. Furthermore, the sliding piece 3222 in direct contact with the blood is received in the receiving cavity of the rotary knob 3221, and the doctor rotates the rotary knob 3221 to move the sliding piece 3222 in the moving channel 3212, thereby preventing the external environment from contaminating the blood in the moving channel 3212.

In other embodiments, a protrusion is formed on an outer wall of the rotary knob 3221, an annular groove into which the protrusion is plugged is formed in an inner wall of the moving channel 3212. An annular groove is formed in an outer wall of the moving channel 3212, and a protrusion plugged into the annular groove is formed on the rotary knob 3221. As long as the rotary knob 3221 rotates along the moving channel 3211 but cannot move in the axial direction. In other embodiments, a cavity is formed at a distal end of the sliding piece 3222, and a distal end of the rotary knob 3221 is inserted into the cavity and is in threaded connection with the sliding piece 3222. It should be noted that the "proximal end" refers to an end that is close to an operator of the flow control element in the axial direction, and the "distal end" refers to an end that is away from the operator of the flow control element in the axial direction.

Referring to FIG. 2. In this embodiment, the blood filter 33 includes a filter element 331 and a filter screen 332 arranged in the filter element 331. The filter element 331 includes a tubular structure with openings at two ends, and a gap is provided between the filter screen 332 and the filter element 331. The filter screen 332 includes a semi-closed structure with an opening at one end, and the opening of the filter screen 332 is connected to the outflow end of the flow controller 32. The filter screen 332 is made from a woven material with meshes. After the blood flows out of the flow controller 32 and enters the filter screen 332, the thrombus is blocked in the filter screen 332, and the filtered blood flows out of the filter screen 332. Because the filter screen is of a semi-closed structure with an opening at one end, the blood can flow out of the filter screen 332 in any direction. Even if the filter screen 332 is partially blocked by the thrombus, other parts of the filter screen 332 still have a filtering function to prevent the extracorporeal circulation system from being blocked during surgery.

The extracorporeal circulation system of this embodiment is applied to CAS, and the thrombus may fall off during the entire surgery. Therefore, in this embodiment, the circumference of the minimum cross section of the filter screen 332 is at least twice the circumference of the maximum cross section of the flow-through channel 3211. In this way, the filter screen 332 has a large filter area, thereby preventing the thrombus from completely blocking the filter screen 332. Preferably, the circumference of the minimum cross section of the filter screen 332 is 2.5 times the body section 32113 of the flow-through channel 3211, to avoid an excessively small gap between the filter screen 332 and the filter element 331 due to an excessively large circumference of the minimum cross section of the filter screen 332.

Based on this, as shown in FIG. 5, the flow-through channel 3211 further includes a flared section 32114 arranged behind the body section 32113. An inner diameter of the flared section 32114 gradually increases from the inflow end 3211a to the outflow end 3211b, to connect the flow-through channel 3211 and the filter screen 332.

In other embodiments, the blood filter may include a plurality of filter screens arranged at intervals in the axial direction from an inflow direction to an outflow direction of the blood filter. Diameters of meshes of the plurality of filter screens gradually increase, to implement multi-layer filtration of the blood, thereby enhancing the filtering effect of the extracorporeal circulation system.

The one-way check valve 34 may be any type of check valve applied to a medical device, which allows the fluid to flow in only one direction. Examples of suitable check valves include, but are not limited to, a seal ring valve, a protector valve, a ball check valve, a diaphragm check valve, a swing check valve, a band check valve, a lift check valves, a duckbill valve, and the like.

Referring to FIG. 2, the blood filter has a filter outlet channel 3311. The filter outlet channel 3311 is connected to the one-way check valve 34. The inner diameter of the filter outlet channel 3311 is denoted as d, the inner diameter of the blood filter is denoted as D, and d≤1/4D. In this embodiment, d=3/16D. When the blood flows through the blood filter 33, because the diameter of the blood filter 33 is large, the flow velocity of the blood is significantly reduced. However, the one-way check valve is mainly opened by an impact force of the blood flow. By arranging the filter outlet channel 3311 with a small inner diameter, the flow velocity of the blood is increased. Therefore, the blood applies a large impact force to the one-way check valve, to successfully flow through the one-way check valve. Meanwhile, the high blood flow velocity can further avoid blood backflow.

The extracorporeal circulation system further has a thermal insulation function. Specifically, in this embodiment, the exterior of the inflow tube 2 and the exterior of an outflow tube 4 are both covered with a thermal insulation material, such as a rubber-plastic insulation tube or a foam insulation tube. In other embodiments, a heating apparatus may be further arranged at the inflow tube 2, the outflow tube 4, or the venous sheath 5 to maintain the blood at a constant temperature.

### Embodiment 2

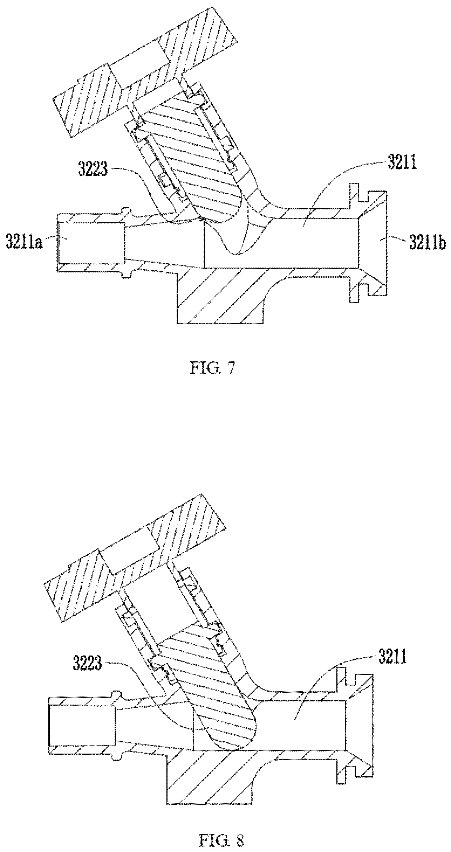

A difference between the flow controller 32 of Embodiment 2 and the flow controller 32 of Embodiment 1 is that: as shown in FIG. 6 and FIG. 7, the included angle between the central axis of a flow-through channel 3211 and the central axis of a moving channel 3212 is an acute angle, the moving channel 3212 is inclined toward the inflow end of the body element 321, and the partial peripheral surface of the flow control element 322 forms a flow guiding surface 3223. Specifically, a distal end of the flow control element 322 is in a cylinder-like shape. When the distal end of the flow control element 322 extends into the flow-through channel 3211 to block the flow-through channel 3211, the blood flows along the peripheral surface of the flow control element 322. The peripheral surface of the flow control element 322 is inclined relative to a blood flow direction and is a curved surface, so that it is difficult for thrombi to deposit, and the unblocked extracorporeal circulation system is ensured.

Preferably, the flow control element 322 includes a rotary knob 3221 and a sliding piece 3222. The rotary knob 3221 is rotatably arranged at a proximal end of the moving channel 3212, a receiving cavity is formed at the distal end of the rotary knob 3221, and the sliding piece 3222 is arranged in the receiving cavity. The sliding piece 3222 has a protrusion extending in a radial direction, and the protrusion extends through a through slot on a side wall of the receiving cavity and is in threaded connection with the moving channel 3212. When the rotary knob 3221 rotates under an external force, the protrusion abuts against the through slot, so that the sliding piece 3222 rotates together. Because the sliding piece 3222 is in threaded connection with the moving channel 3212, the sliding piece 3222 spirally moves along the moving channel with the rotation of the rotary knob 3221. The sliding piece 3222 in direct contact with the blood is received in the receiving cavity of the rotary knob 3221, and a doctor rotates the rotary knob 3221 to move the sliding piece 3222 in the moving channel 3212, thereby preventing the external environment from contaminating the blood in the moving channel 3212.

Preferably, the entire sliding piece 3222 is in a cylindrical shape, with a diameter equal to the diameter of the flow-through channel 3211. The terminal of the sliding piece 3222 is in the shape of a ball head, and the diameter of the terminal in the shape of a semi-ball head is equal to the inner diameter of the flow-through channel 3211. Based on this, as shown in FIG. 8, the distal end of the sliding piece can closely fit the flow-through channel 3211, so that the flow controller 32 can function to block the blood flow. The peripheral surface of the sliding piece 3222 forms the flow guiding surface. It should be noted that the peripheral surface includes a curved surface coaxial with the central axis of the sliding piece 3222, and further includes a curved surface of a distal end portion of the sliding piece 3222.

### Embodiment 3

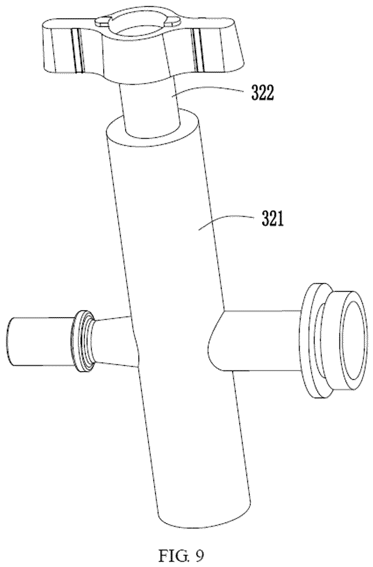

A difference between the flow controller 32 of Embodiment 3 and the flow controller 32 of Embodiment 1 is that: as shown in FIG. 9 to FIG. 11, a plurality of communication holes 3224 are formed in a flow control element 322, and the communication holes 3224 extend in the same direction as the flow-through channel 3211. At least one communication hole 3224 includes a velocity regulating section 32241. The entire velocity regulating section 32241 is in the shape of a hopper gradually decreasing from the inflow end 3211a to the outflow end 3211b. The inner wall of the velocity regulating section 32241 forms a flow guiding surface 3223. The edge of the inflow end of the velocity regulating section 32241 may be in smooth transition connection with the inner wall of the flow-through channel 3211.

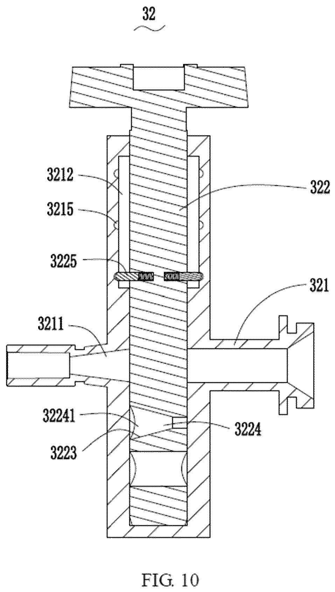

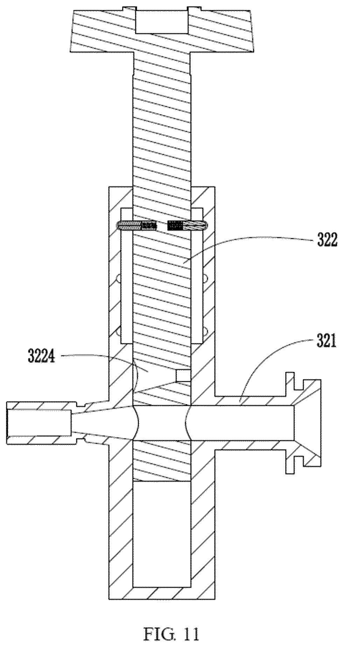

A plurality of communication holes 3224 with different diameters are formed in the flow control element 322, as shown in FIG. 10 and FIG. 11. When the flow control element moves along the moving channel 3212 under an external force, the blood flows through the communication holes 3224 with different diameters, thereby regulating the blood flow rate to different extents. When all the communication holes do not face the flow-through channel, the flow-through channel is blocked, and the flow controller is in an off state. Because the inflow end of the velocity regulating section 32241 may be in smooth transition connection with the inner wall of the flow-through channel 3211, it is difficult for thrombi to deposit at the flow control element 322.

A plurality of first matching portions 3215 are arranged on the body element 321, a plurality of second matching portions 3225 are arranged on the flow control element 322, and when any first matching portion 3215 is matched with the second matching portion 3225, the edge of the inflow end of the corresponding velocity regulating section 32241 is in smooth transition connection with the inner wall of the flow-through channel 3211. In this embodiment, the first matching portion 3215 is an arc-shaped hole in the inner wall of the flow-through channel 3211, the second matching portion 3225 is a spring block that can be extended and retracted in a radial direction of the flow control element 322, and a terminal of the spring block is arc-shaped. When the spring block is clamped in the arc-shaped hole, the communication hole 3224 faces the flow-through channel 3211, and the edge of the inflow end of the corresponding velocity regulating section 32241 is in smooth transition connection with the inner wall of the flow-through channel 3211. When the flow control element 322 moves along the moving channel 3212 under an external force, the spring block is squeezed back into the flow control element 322 until the spring block reaches the next arc-shaped hole. By adopting the foregoing structure, a doctor can accurately operate the flow control element 322, to ensure that the communication hole 3224 faces the flow-through channel 3211.

### Embodiment 4

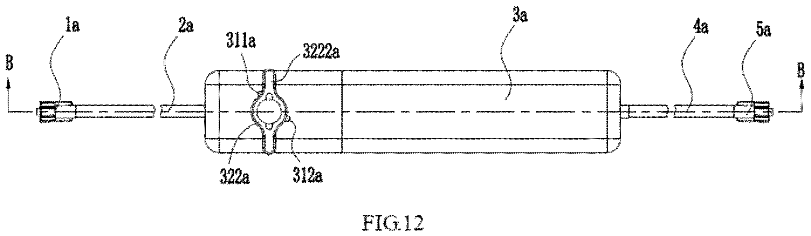

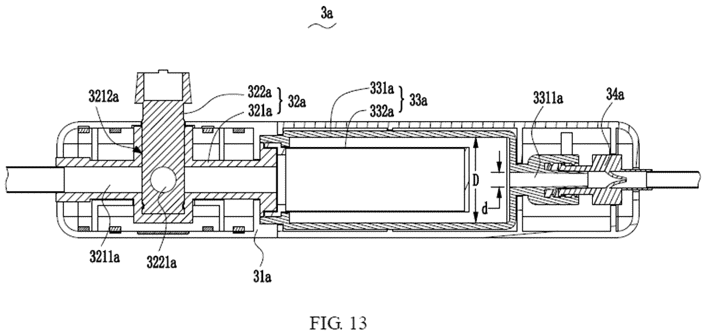

As shown in FIG. 12 and FIG. 13, an extracorporeal circulation system of this embodiment includes an arterial sheath 1a, an inflow tube 2a, a handle 3a, an outflow tube 4a, and a venous sheath 5a that are connected in sequence. The arterial sheath 1a is connected to a carotid artery, and the venous sheath 5a is connected to a vein.

The handle 3a includes a housing 31a, as well as a flow controller 32a, a blood filter 33a, and a one-way check valve 34a that are arranged in the housing 31a and are communicated in sequence. The flow controller 32a is connected to the end of the inflow tube 2a that is away from the arterial sheath 1a, and the one-way check valve 34a is connected to the end of the outflow tube 4a that is away from the venous sheath 5a.

Before a stent is placed at a stenotic site of the carotid artery, the arterial sheath 1a of this embodiment is placed in the common carotid artery and the venous sheath 5a is placed in the femoral vein. A pressure difference is formed between the high-pressure arterial blood flow in the brain and the low-pressure venous blood flow in the femoral vein, so that the blood in the carotid artery flows in reverse to the femoral vein through the extracorporeal circulation system. After the reverse blood circulation system is established, operations, such as pre-expansion of the stenotic site of the carotid artery and placement of a self-expanding stent, are performed. A thrombus falling off during surgery flows with the reverse blood flow and is intercepted by the filter, so that the thrombus is prevented from entering the human body with the blood flow. A doctor can steplessly regulate a flow rate of the reverse blood flow in real time by using the flow controller 32.

In this embodiment, as shown in FIG. 13, the flow controller 32a includes a body element 321a and a flow control element 322a. A flow-through channel 3211a and a moving channel 3212a are arranged in the body element. The flow control element 322a is rotatably arranged in the moving channel 3212a. A circulation hole 3221a is formed in the flow control element 322a and is communicated with the flow-through channel 3211a. When the flow control element 322a rotates under an external force, the position of the circulation hole 3221a on the flow control element 322 is changed. The area of a surface that is communicated with the flow-through channel 3211a of the through hole 3221a is changed from large to small or from small to large. In this way, the blood flow rate can be regulated steplessly by the flow controller 32a to avoid causing discomfort to the human body due to sudden changes in the blood flow.

Referring to FIG. 12, a first limiting piece 311a and a second limiting piece 312a are arranged outside the housing 31a. When the flow control element 322a abuts against the first limiting piece 311a, the flow controller 32a is in an off state; and when the flow control element 322a abuts against the second limiting piece 312a, the flow control element 32a is in maximum flow state. In this embodiment, the flow control element 322a has a handle 3222a extending in a radial direction. The handle 3222a may abut against the first limiting piece 311a or the second limiting piece 312a, to prevent the flow control element 322a from continuing to rotate. Therefore, when the flow control element 322a of this embodiment rotates in the same direction, the flow rate can only be regulated from small to large or from large to small, which is convenient for a doctor to operate. In other embodiments, the first limiting piece and the second limiting piece may be arranged inside the housing, and a corresponding structure that can abut against the first limiting piece and the second limiting piece is arranged on the flow control element.

Preferably, the flow control element 322a abuts against a bottom surface of the flow-through channel 3211a. The flow control element 322a abuts against the bottom surface of the flow-through channel 3211a, so that a friction force between the flow control element 322a and the body element 321a can be increased, and the resistance to rotation of the flow control element 322a can be increased. In this way, the flow control element 322a is prevented from passively rotating under the flushing of the blood flow.

As shown in FIG. 13, in this embodiment, the filter 33a includes a filter element 331a and a filter screen 332a arranged in the filter element 331a. The filter element 331a includes a tubular structure with openings at two ends, and a gap is provided between the filter screen 332a and the filter element 331a. The filter screen 332a includes a semi-closed structure with an opening at one end, and the opening of the filter screen 332a is connected to an outflow end of the flow controller 32a. The filter screen 332a is made from a woven material with meshes. After the blood flows out of the flow controller 32a and enters the filter screen 332a, the thrombus is blocked in the filter screen 332a, and the filtered blood flows out of the filter screen 332a. Because the filter screen includes the semi-closed structure with an opening at one end, the blood can flow out of the filter screen 332a in any direction. Even if the filter screen 332a is partially blocked by the thrombus, other parts of the filter screen 332a still have a filtering function to prevent the extracorporeal circulation system from being blocked during surgery.

The extracorporeal circulation system of this embodiment is applied to CAS, and the thrombus may fall off during entire surgery. Therefore, in this embodiment, the circumference of the minimum cross section of the filter screen 332a is at least twice that of the flow-through channel 3211a. In this way, the filter screen 332a has a large filter area, thereby preventing the thrombus from completely blocking the filter screen 332a. Preferably, the circumference of the minimum cross section of the filter screen 332a is 2.5 times that of the flow-through channel 3211a, to avoid an excessively small gap between the filter screen and the filter element 331a due to an excessively large circumference of the filter screen 332a.

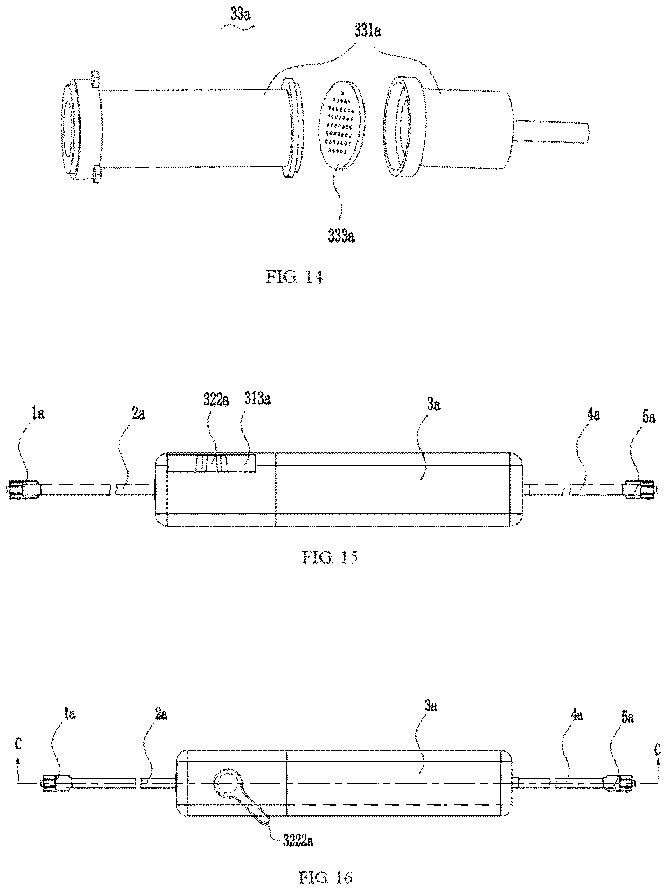

In other embodiments, as shown in FIG. 14, the filter 33a includes a filter element 331a and at least one sheet-like filter screen 333a arranged in the filter element 331a. The filter element 331a includes a tubular structure with openings at two ends, and the sheet-like filter screen 333a is perpendicular to the central axis of the filter element. In the filter with a plurality of sheet-like filter screens 333a, the mesh number of the plurality of sheet-like filter screens 333a is gradually increased from the inflow direction to the outflow direction of the filter 33a, to implement multi-layer filtration of the blood. The sheet-like filter screen 333a with small mesh number can cut off a large thrombus, thereby avoiding blocking due to direct filtration by the sheet-like filter screen with large mesh number.

The one-way check valve 34a may be any type of check valve applied to a medical device, which allows the fluid to flow in only one direction. Examples of suitable check valves include, but are not limited to, a seal ring valve, a protector valve, a ball check valve, a diaphragm check valve, a swing check valve, a band check valve, a lift check valves, a duckbill valve, and the like.

Referring to FIG. 13, the blood filter has a filter outlet channel 3311a. The filter outlet channel 3311a is connected to the one-way check valve 34a. The inner diameter of the filter outlet channel 3311a is denoted as d, the inner diameter of the blood filter is denoted as D, and d≤1/4D. In this embodiment, d=3/16D. When the blood flows through the blood filter 33a, because the diameter of the blood filter 33a is large, the flow velocity of the blood is significantly reduced. However, the one-way check valve is mainly opened by an impact force of the blood flow. By arranging the filter outlet channel 3311a with a small inner diameter, the flow velocity of the blood is increased. Therefore, the blood applies a large impact force to the one-way check valve, to successfully flow through the one-way check valve. Meanwhile, the high blood flow velocity can further avoid blood backflow.

The extracorporeal circulation system further has a thermal insulation function. Specifically, in this embodiment, the exterior of the inflow tube 2a and the exterior of an outflow tube 4a are both covered with a thermal insulation material, such as a rubber-plastic insulation tube or a foam insulation tube. In other embodiments, a constant heating apparatus may be further arranged at the outflow tube 4a or the venous sheath 5a to maintain the blood at a constant temperature.

### Embodiment 5

A difference between the handle of Embodiment 5 and the handle of Embodiment 4 is mainly that: as shown in FIG. 15, a window 313a is formed in a side surface of a housing 31a, a flow control element 322a is mainly arranged in the housing 31a, and a part of the flow control element 322a is exposed outside the housing from the window 313a. By arranging the window 313a, a doctor can hold the handle 3a with one hand, and directly moves the part that is exposed outside the window 313a of the flow control element 322a with the thumb. In this way, the doctor can quickly and conveniently regulate the blood flow rate of an extracorporeal circulation system.

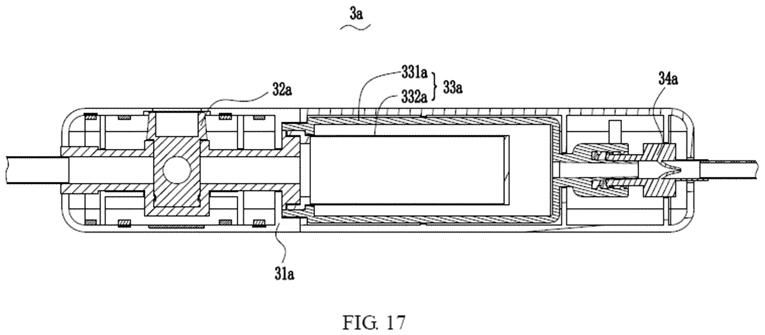

In this embodiment, as shown in FIG. 16 and FIG. 17, a handle 3222a is arranged on the flow control element 322a. The handle 3222a is exposed outside the housing 31a from the window 313a. For ease of understanding, the dotted line in FIG. 16 represents a structure that is located in the housing 31a of the flow control element 322a. When the handle 3222a abuts against one side of two sides of the window 313a, the flow controller is in an off state or a maximum flow state. The handle 3222a is not only convenient for the doctor to regulate the flow rate with one hand, but also can be used as a limiting structure. Therefore, the blood flow rate can be regulated from small to large or from large to small by moving the handle 3222a in the same direction without the need of an additional limiting structure. In other embodiments, the top of the flow control element 322a may be configured as a roller coaxial with a moving channel, and the roller is partially exposed outside the window 313a.

The technical features of the foregoing embodiments may be combined in different manner to form other embodiments. For ease of description, not all possible combinations of the technical features in the foregoing embodiments are described. However, the combinations of these technical features are considered as falling within the scope recorded by the description provided that no conflict exists.

The foregoing embodiments only describe several implementations of the present invention, which are described specifically and in detail, but cannot be construed as a limitation to the patent scope of the present invention. It should be noted that for those of ordinary skill in the art, several transformations and improvements may be made without departing from the idea of the present invention. These transformations and improvements shall fall within the scope of protection of the present invention. Therefore, the scope of protection of the present invention is subject to the appended claims.

## Claims

1. An extracorporeal circulation system, **characterized by** comprising an arterial sheath, an inflow tube, a handle, an outflow tube, and a venous sheath that are connected in sequence, wherein the arterial sheath is used for connecting to a carotid artery, and the venous sheath is used for connecting to a vein;
the handle comprises a housing, as well as a flow controller, a blood filter, and a one-way check valve that are arranged in the housing and are communicated in sequence; and the flow controller is connected to an end of the inflow tube that is away from the arterial sheath, and the one-way check valve is connected to the end of the outflow tube that is away from the venous sheath; and
the flow controller comprises one body element with a flow-through channel and a flow control element, at least part of the flow control element is received in the body element and is movably connected to the flow-through channel, and the flow control element reduces or increases the circulation area of the flow-through channel under an external force.

2. The extracorporeal circulation system according to claim 1, **characterized in that**
the flow-through channel has an inflow end and an outflow end, the inflow end is close to the inflow tube, and the outflow end is far away from the inflow tube; and
the flow control element has one flow guiding surface, the flow guiding surface faces the inflow end, and one flow regulating channel gradually decreasing from the inflow end to the outflow end is defined by the flow guiding surface and an inner surface of the flow-through channel.

3. The extracorporeal circulation system according to claim 2, **characterized in that**
a moving channel communicated with the flow-through channel is further arranged in the body element; and
the flow control element is movably arranged in the moving channel.

4. The extracorporeal circulation system according to claim 1, **characterized in that**
the flow-through channel comprises a connecting section, a narrowed section, and a body section that are connected in sequence from the inflow end to the outflow end; and the inner diameter of the narrowed section is smaller than inner diameters of any one of the connecting section and the body section.

5. The extracorporeal circulation system according to claim 3, **characterized in that**
one inclined surface is formed at a distal end of the flow control element and forms the flow guiding surface.

6. The extracorporeal circulation system according to claim 5, **characterized in that**
a distal end surface of the flow control element is matched with the shape of the bottom of the flow-through channel, and when the flow controller is in an off state, the distal end surface of the flow control element fits the bottom of the flow-through channel.

7. The extracorporeal circulation system according to claim 5, **characterized in that**
the flow control element comprises a rotary knob and a sliding piece, and the sliding piece is slidably arranged in the moving channel; and the rotary knob is rotatably connected to the body element and is in threaded connection with the sliding piece, and the rotary knob rotates under an external force and drives the sliding piece to spirally move in the moving channel; and
the inclined surface is arranged at a distal end of the sliding piece.

8. The extracorporeal circulation system according to claim 3, **characterized in that**
an included angle between the central axis of the flow-through channel and the central axis of the moving channel is an acute angle, the moving channel is inclined toward the inflow end of the body element, and a partial peripheral surface of the flow control element defines the flow guiding surface.

9. The extracorporeal circulation system according to claim 8, **characterized in that**
a terminal of the flow control element is in the shape of a ball head, and the diameter of the terminal in the shape of the ball head is equal to the inner diameter of the flow-through channel.

10. The extracorporeal circulation system according to claim 3, **characterized in that**
a plurality of communication holes are formed in the flow control element, the communication holes extend in the same direction as the flow-through channel, at least one communication hole includes a velocity regulating section, the entire velocity regulating section is in the shape of a hopper gradually decreasing from the inflow end to the outflow end, and an inner wall of the velocity regulating section defines the flow guiding surface; and
an edge of an inflow end of the velocity regulating section is in smooth transition connection with an inner wall of the flow-through channel.

11. The extracorporeal circulation system according to claim 10, **characterized in that**
a plurality of first matching portions are arranged on the body element, a plurality of second matching portions are arranged on the flow control element, and when any first matching portion is matched with the second matching portion, the edge of the inflow end of the corresponding velocity regulating section is in smooth transition connection with the inner wall of the flow-through channel.

12. The extracorporeal circulation system according to claim 1, **characterized in that**
a moving channel communicated with the flow-through channel is further arranged in the body element, the flow control element is rotatably arranged in the moving channel, and a circulation hole is formed in the flow control element and is communicated with the flow-through channel.

13. The extracorporeal circulation system according to claim 12, **characterized in that**
a first limiting piece and a second limiting piece are arranged outside the housing; when the flow control element abuts against the first limiting piece, the flow controller is in an off state; and when the flow control element abuts against the second limiting piece, the flow controller is in a maximum flow state.

14. The extracorporeal circulation system according to claim 12, **characterized in that**
the flow control element abuts against a bottom surface of the moving channel.

15. The extracorporeal circulation system according to claim 12, **characterized in that**
a window is formed in a side surface of the housing, and the flow control element is mainly arranged in the housing; and
a handle is arranged on the flow control element and is exposed outside the housing from the window, and when the handle abuts against one side of two sides of the window, the flow controller is in an off state or a maximum flow state.

16. The extracorporeal circulation system according to claim 1, **characterized in that**
the blood filter comprises a filter element and a filter screen arranged in the filter element; the filter element comprises a tubular structure with openings at two ends, and a gap is provided between the filter screen and the filter element; and the filter screen comprises a semi-closed structure with an opening at one end, and the opening of the filter screen is connected to the outflow end of the flow controller.

17. The extracorporeal circulation system according to claim 16, **characterized in that**
the circumference of the minimum cross section of the filter screen is at least twice the circumference of the cross section of the flow-through channel.

18. The extracorporeal circulation system according to claim 1, **characterized in that**
the filter comprises a filter element and at least one sheet-like filter screen arranged in the filter element; and the filter element comprises a tubular structure with openings at two ends, and the sheet-like filter screen is perpendicular to the central axis of the filter element.

19. The extracorporeal circulation system according to claim 1, **characterized in that**
the inflow tube and the outflow tube are made from a thermal insulation material, or the exterior of the inflow tube and the exterior of the outflow tube are covered with a thermal insulation layer, or a heating apparatus is arranged at the outflow tube or the venous sheath.

20. The extracorporeal circulation system according to claim 1, **characterized in that**
the blood filter has a filter outlet channel, the filter outlet channel is connected to the one-way check valve, the inner diameter of the filter outlet channel is denoted as d, the inner diameter of the blood filter is denoted as D, and d≤1/4D.
